# EUROPEAN PATENT APPLICATION

(11) **EP 4 768 069 A1**
(43) Date of publication of application: **01.07.2026**
(21) Application number: 24879044.6
(22) Date of filing: 16.10.2024
(51) Int. Cl.: A61M 60/816, A61M 60/40, A61M 60/00

(54) **DRIVING APPARATUS AND BLOOD PUMP**

(30) Priority: 19.10.2023 CN 202311357284
(71) Applicant: Shenzhen Core Medical Technology Co., Ltd., Shenzhen, Guangdong 518000 (CN)
(72) Inventor: ZHANG, Qi, Shenzhen, Guangdong 518000 (CN); SHEN, Rui, Shenzhen, Guangdong 518000 (CN); SU, Zhou, Shenzhen, Guangdong 518000 (CN); YU, Shunzhou, Shenzhen, Guangdong 518000 (CN)
(74) Representative: Appleyard Lees IP LLP
(86) International application number: PCT/CN2024/125279
(87) International publication number: WO 2025/082403

(57) **Abstract**

A driving apparatus and a blood pump. The driving apparatus comprises a motor (30), an outer sealing tube (20), and a detection assembly (10), wherein the motor (30) comprises a motor housing (31), and a detection port (311) is formed on the motor housing (31). The outer sealing tube (20) is arranged in the motor housing (31) and is fixedly connected to the motor housing (31). A side opening (21) is formed on an outer side surface of the outer sealing tube (20) and is arranged opposite to the detection port (311). The detection assembly (10) penetrates through the outer sealing tube (20), and comprises a sensor (11) and an inner sealing tube (12), the inner sealing tube (12) being fixed in the outer sealing tube (20). A distal end of the inner sealing tube (12) extends to an inner side of the side opening (21) and is provided with a sensing port (122) corresponding to the side opening (21). A probe (111) of the sensor (11) and a portion of an optical fiber (112) connected to the probe (111) are encapsulated within the inner sealing tube (12), the probe (111) corresponding to the sensing port (122).

## Description

This application claims priority to Chinese Patent Application No. CN202311357284.5, entitled "DRIVING DEVICE AND BLOOD PUMP", filed with China National Intellectual Property Administration on October 19, 2023, the entire content of which is incorporated herein by reference.

### TECHNICAL FIELD

The present application relates to the field of medical apparatus and instrument technologies, and in particular, to a driving device and a blood pump.

### BACKGROUND

A driving device of a blood pump is usually provided with a sensor for detecting a blood pressure. In order to avoid contamination of the sensor by blood or a perfusion irrigation fluid, the sensor is usually required to be packaged.

In the related art, the sensor in the driving device is arranged in a rubber tube, the rubber tube is inserted into a motor housing of the driving device, and meanwhile, a proximal end of the sensor is fixed to the rubber tube by dispersing a hard adhesive, and a distal end of the sensor is fixed to the rubber tube and the motor housing by dispersing a soft adhesive. However, the soft adhesive at the distal end of the sensor is prone to loosen due to washing by the blood, and since a diameter of the sensor is smaller than an inner diameter of the rubber tube, after the soft adhesive at the distal end of the sensor loosens, the sensor is quite prone to jump along an axial direction of the rubber tube, resulting in inaccurate detection.

### SUMMARY

Accordingly, it is necessary to a driving device and a blood pump to address the problem that packaging stability of a sensor is poor and detection precision is influenced in the conventional blood pump.

In an embodiment of the present application, the driving device includes a motor, an outer packaging tube and a detection assembly. The motor includes a motor housing, and is provided with a detection port. The outer packaging tube is inserted into the motor housing along an axial direction of the motor, the outer packaging tube is fixedly connected to the motor housing, a distal end of the outer packaging tube being provided with a side opening opposite to the detection port. The detection assembly includes a sensor and an inner packaging tube, wherein the inner packaging tube is fixed inside the outer packaging tube, and a distal end of the inner packaging tube extends to an inner side of the side opening and is provided with a sensing port corresponding to the side opening; the sensor has a probe and an optical fiber connected to the probe, the probe and a part of the optical fiber are packaged in the inner packaging tube, and the probe corresponds to the sensing port.

In an embodiment of the blood pump according to the present application, the blood pump includes a catheter and a driving device.

The driving device includes:
a motor including a motor housing, and the motor housing being provided with a detection port;
an outer packaging tube inserted into the motor housing along an axial direction of the motor, the outer packaging tube being fixedly connected to the motor housing, and a distal end of the outer packaging tube being provided with a side opening opposite to the detection port; and
a detection assembly, the detection assembly including a sensor and an inner packaging tube, wherein the inner packaging tube is fixed inside the outer packaging tube, and a distal end of the inner packaging tube extends to an inner side of the side opening and is provided with a sensing port corresponding to the side opening; the sensor has a probe and an optical fiber connected to the probe, the probe and a part of the optical fiber are packaged in the inner packaging tube, and the probe corresponds to the sensing port.

Te catheter is connected to the driving device, and the optical fiber of the sensor penetrates out of the catheter.

Details of one or more embodiments of the present invention are set forth in the accompanying drawings and the description below. Other features, objects, and advantages of the present invention will be apparent from the description, the accompanying drawings, and the claims.

### BRIEF DESCRIPTION OF THE DRAWINGS

In order to illustrate the technical solutions in the embodiments of the present application more clearly, the drawings required for describing the embodiments or the prior art will be described briefly. Apparently, the following described drawings are merely for some embodiments of the present application, and other drawings can be derived from these drawings by those of ordinary skill in the art without any creative effort.
FIG. 1 is a schematic view of a blood pump according to an embodiment.
FIG. 2 is a sectional view of the blood pump shown in FIG. 1 taken along line A-A.
FIG. 3 is a partial enlarged view of portion P₁ shown in FIG. 2.
FIG. 4 is a sectional view of the blood pump shown in FIG. 1 taken along line B-B.
FIG. 5 is a partial enlarged view of portion P₂ shown in FIG. 4.
FIG. 6 is a partial sectional view of a driving device according to an embodiment.
FIG. 7 is a sectional view of a detection assembly in an embodiment.
FIG. 8 is a schematic diagram of structural division of a sensor of the driving device according to an embodiment.
FIG. 9 is a schematic size diagram of one part of a structure of the driving device according to an embodiment.
FIG. 10 is a schematic size diagram of the other part of the structure of the driving device according to an embodiment.
FIG. 11 is a partial sectional view of the driving device according to another embodiment.
FIG. 12 is a schematic view of the detection assembly in an embodiment.
FIG. 13 is a schematic view of an outer packaging tube in an embodiment.

### DETAILED DESCRIPTION

The present application will be described in further detail below with reference to the accompanying drawings and embodiments in order to make the objects, technical solutions, and advantages of the present application more clear. It should be understood that the specific embodiments described herein are only for explaining the present application, and not intended to limit the present application.

It should be noted that when an element is referred to as being "fixed on" or "provided at" another element, the element may be directly or indirectly located on the other element. When an element is referred to as being "connected to" another element, the element may be directly or indirectly connected to the other element.

In addition, the terms such as "first" and "second" are used herein for purposes of description and are not intended to indicate or imply relative importance or significance or to imply the number of indicated technical features. Thus, the feature defined with "first" and "second" may include one or more of this feature explicitly or implicitly. In the description of the present application, "a plurality of" means two or more, unless otherwise specified.

In order to explain the technical solution of the present application, the following description is given with reference to the specific drawings and embodiments.

It should be first noted that, in the embodiment of the present application, the "distal end" refers to the end away from an operator during an operation, and the "proximal end" refers to the end adjacent to the operator during the operation.

In the related art, a detection port is formed in a distal end of a motor housing of a motor of a traditional blood pump, a sensor is packaged in the motor housing, and a distal end of the sensor corresponds to the detection port, so as to detect blood parameters through the detection port. A packaging method of the sensor of the traditional blood pump generally includes: forming an insertion port in a proximal end of the motor housing, then inserting the sensor into a rubber tube, inserting the rubber tube with the sensor into the motor housing of the motor from the insertion port, and finally dispensing adhesives at the detection port and the insertion port respectively to fix the two ends of the sensor to the rubber tube or the motor housing. That is, in the traditional sensor packaging method, the two ends of the sensor are only fixedly bonded to the detection port and the insertion port by the adhesives.

However, the adhesive at the detection port is in contact with blood flow and is prone to loosen due to washing by the blood flow; the adhesive at the insertion port may also loosen due to aging. Once the adhesive at one of the detection port and the insertion port loosens, the sensor may loosen, and since a diameter of an optical fiber of the sensor is usually smaller than an inner diameter of the rubber tube, after the sensor loosens due to loosening of the adhesive, the sensor is quite prone to jump along an axial direction of the rubber tube, so that packaging firmness of the sensor is poor, and detection of the sensor is prone to be inaccurate.

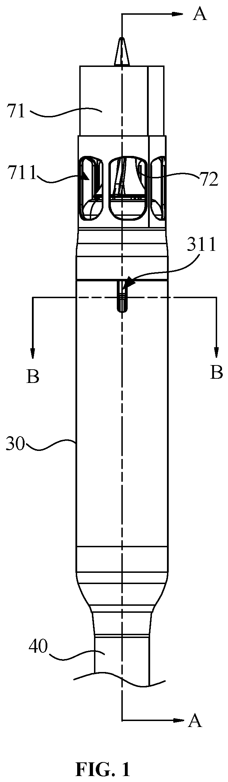

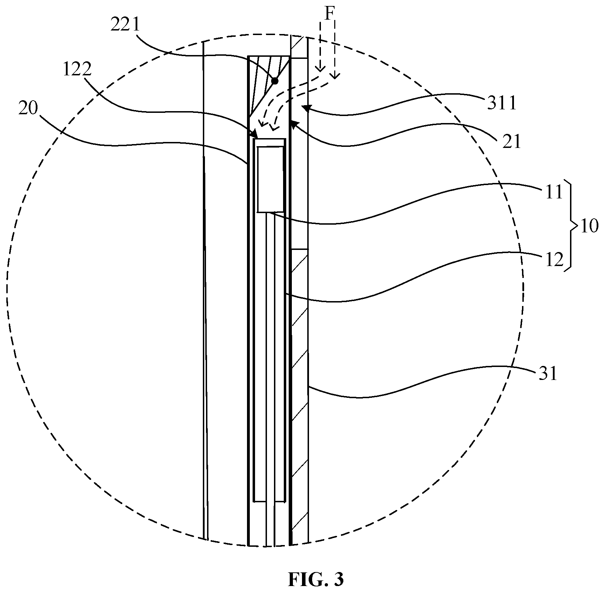

In view of this, referring to FIG. 1 to FIG. 3, an embodiment of the present application provides a driving device and a blood pump including the driving device. The driving device is configured to drive an impeller 70 of the blood pump to rotate, so as to drive blood to flow. For the sake of understanding, in the following embodiments, the driving device is described first.

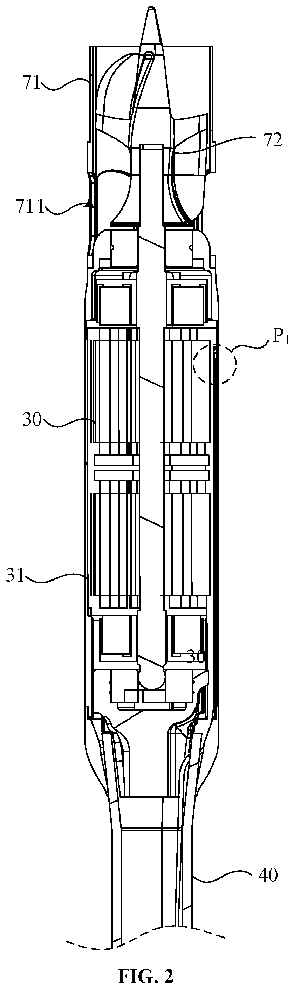

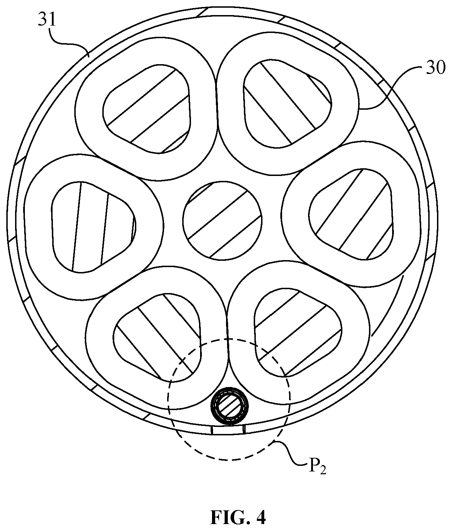

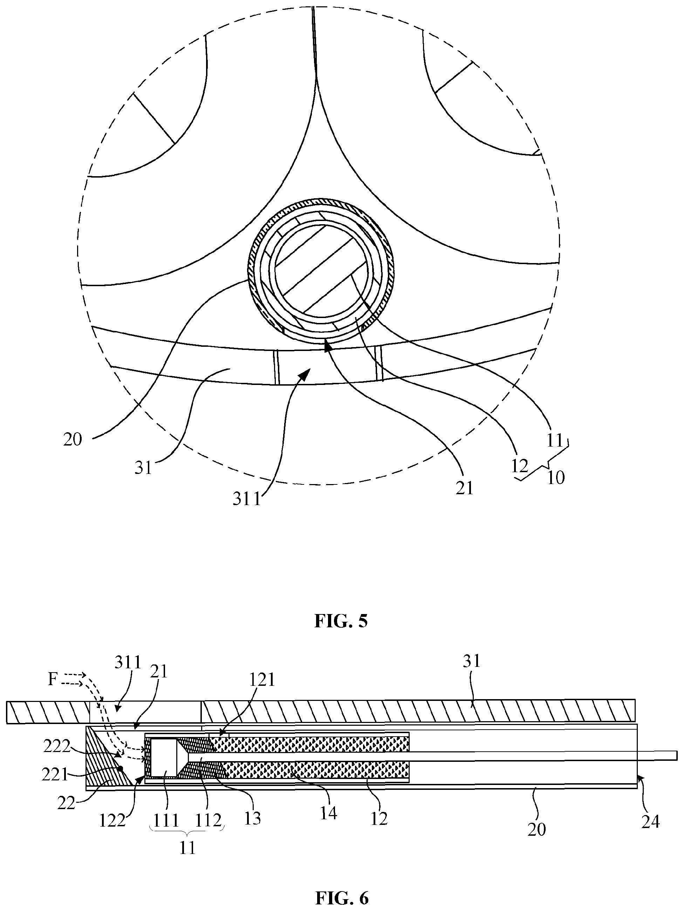

Referring to FIG. 2 to FIG. 5, in an embodiment, the driving device includes a motor 30, an outer packaging tube 20, and a detection assembly 10. The motor 30 includes a motor housing 31, and the motor housing 31 is provided with a detection port 311 for the blood to flow in. The outer packaging tube 20 is inserted into the motor housing 31 along an axial direction of the motor 30 and fixedly connected to the motor housing 31. A distal end of the outer packaging tube 20 is provided with a side opening 21, which is opposite to the detection port 311. With reference to FIG. 6, the detection assembly 10 is inserted into the outer packaging tube 20. The detection assembly 10 includes a sensor 11 and an inner packaging tube 12. The inner packaging tube 12 is fixed in the outer packaging tube 20, and a distal end of the inner packaging tube 12 extends to an inner side of the side opening 21 and is provided with a sensing port 122 corresponding to the side opening 21. The sensor 11 includes a probe 111 and an optical fiber 112 connected to the probe 111. The probe 111 and a part of the optical fiber 112 are packaged in the inner packaging tube 12, and the probe 111 corresponds to the sensing port 122 to sense the blood through the sensing port 122.

As for the motor housing 31, the detection port 311 of the motor housing 31 may be arranged in a distal end of the motor housing 31, or in a middle portion of the motor housing 31. Specifically, the detection port 311 of the motor housing 31 is arranged in the distal end of the motor housing 31, such that the detection port 311 is adjacent to a blood outlet 711 of the blood pump, therefore a pressure of the blood detected by the detection assembly 10 is closer to a pressure of the blood discharged from the blood outlet 711.

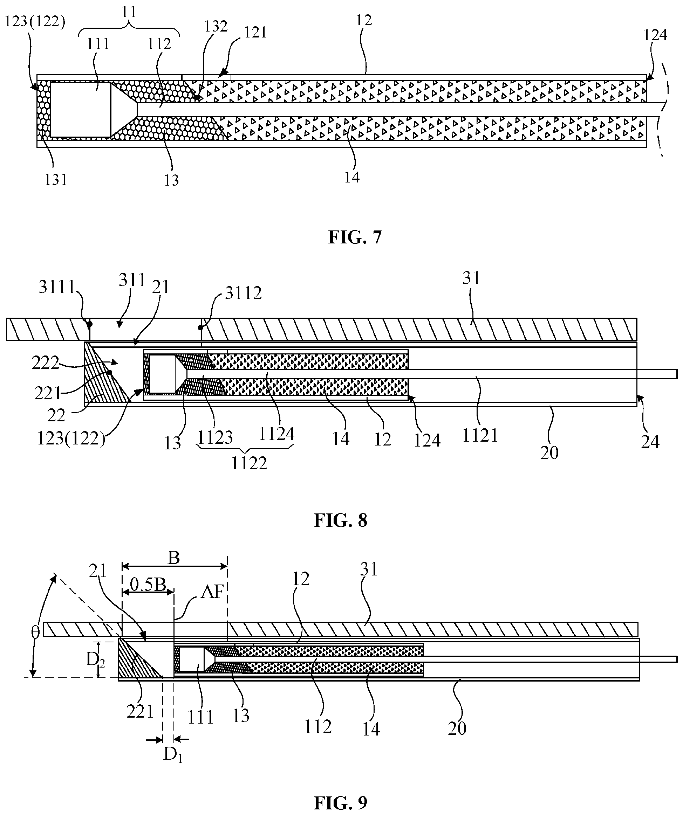

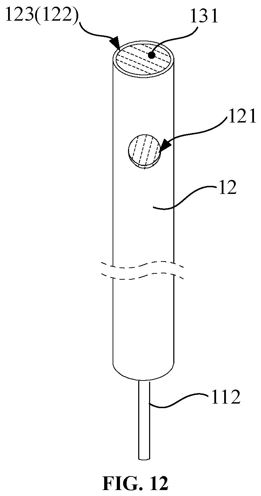

As for the detection assembly 10, packaging the probe 111 and the part of the optical fiber 112 of the sensor 11 in the inner packaging tube 12 means that the probe 111 and the part of the optical fiber 112 are fixed and sealed in the inner packaging tube 12, so that the sensor 11 and the inner packaging tube 12 of the detection assembly 10 are integrated into a whole structure. When the blood pump with the driving device is inserted into a patient, the blood flow F around the blood pump can enter the side opening 21 from the detection port 311, can flow from the side opening 21 to the sensing port 122, and be sensed by the probe 111 of the sensor 11 in the detection assembly 10 at the sensing port 122, so that the sensor 11 can sense the blood pressure. As shown in FIG. 8 and FIG. 12, the inner packaging tube 12 has a distal tube opening 123 and a proximal tube opening 124, and the distal tube opening 123 of the inner packaging tube 12 may serve as the sensing port 122. Certainly, in other embodiments, the distal tube opening 123 of the inner packaging tube 12 can be configured to be closed, andthe sensing port 122 is additionally provided in a side wall of the distal end of the inner packaging tube 12.

In the above driving device, the probe 111 and the part of the optical fiber 112 of the sensor 11 are packaged in the inner packaging tube 12, such that the sensor 11 and the inner packaging tube 12 of the detection assembly 10 are integrated into the whole structure, therefore the sensor 11 is firmly fixed inside the inner packaging tube 12 and is not prone to slide along an axial direction of the inner packaging tube 12. On this basis, the inner packaging tube 12 of the detection assembly 10 is fixed in the outer packaging tube 20, and finally the outer packaging tube 20 is fixed inside the motor housing 31, which is equivalent to packaging of the sensor 11 in a double-tube structure consisting of the inner packaging tube 12 and the outer packaging tube 20. Compared with the conventional single-tube packaging manner for the sensor, in the present application, the sensor 11 is packaged in the inner packaging tube 12 first, and then the inner packaging tube 12 is fixed inside the outer packaging tube 20, and an outer circumferential wall of the inner packaging tube 12 and an inner circumferential wall of the outer packaging tube 20 have larger fitting surfaces, such that the inner packaging tube 12 and the outer packaging tube 20 can be connected and assembled more stably, the inner packaging tube 12 is not prone to move along an axial direction of the outer packaging tube 20, the packaging firmness of the sensor 11 can be further improved, and the sensor 11 is prevented from sliding axially.

Based on the fixation of the outer packaging tube 20 and the motor housing 31, the inner packaging tube 11 may be optionally configured to be in interference fit with the outer packaging tube 20. With such an arrangement, even if an adhesive at the detection port 311 loosens, the detection assembly 10 cannot easily slide in the outer packaging tube 20 due to the interference fit between the inner packaging tube 11 and the outer packaging tube 20, so that the detection assembly 10 can perform detection normally. Further optionally, sealing structures (e.g., sealing caps or sealants) are arranged at both ends of the outer packaging tube 20 to confine the inner packaging tube 11 within the outer packaging tube 20. This arrangement can further improve mounting firmness and a sealing performance of the sensor 11.

It is considered here that, after the blood pump is inserted into the patient, the blood in the blood vessel flows along an axial direction of the blood pump, and when the blood reaches the detection port 311 of the blood pump, a flow direction of a part of the blood switches from the axial direction to a radial direction, this part of the blood turns into the detection port 311 at a deflection angle adjacent to 90°, and the part of the blood turns towards the probe 111 of the sensor 11 at an axial angle of 90° after perpendicularly impacting an inner wall of the outer packaging tube 20, so that the probe 111 of the sensor 11 senses the blood pressure. In this process, the angle of the blood flow is changed too much, and the pressure is reduced to some extent, which may cause a detection result of the detection assembly 10 to be inaccurate.

Referring to FIG. 6 to FIG. 8, in order to solve the above problem, in an embodiment, the detection port 311 has a distal edge 3111 and a proximal edge 3112, and the distal edge 3111 and the proximal edge 3112 are opposite to each other along the axial direction of the motor 30. The inner packaging tube 12 has a distal tube opening 123 configured to form the sensing port 122, and the distal tube opening 123 is located in an inner region between the distal edge 3111 and the proximal edge 3112. The outer packaging tube 20 is provided therein with a flow guide wall 221 located on an inner side of the side opening 21, a flow guide groove 222 is formed between the flow guide wall 221 and the distal tube opening 123 (i.e., the sensing port 122), and the flow guide groove 222 is configured to guide the blood flow from the detection port 311 to the distal tube opening 123.

Specifically, the distal tube opening 123 of the inner packaging tube 12 serves as the sensing port 122. The flow guide wall 221 is located at a distal side of the distal tube opening 123 of the inner packaging tube 12, and the flow guide wall 221 is inclined relative to an axis of the motor 10. After the blood flow passes through the detection port 311 and the side opening 21 in sequence, the blood flow enters the flow guide groove 222 and then smoothly flows along the flow guide wall 221 to the distal tube opening 123 (i.e., the sensing port 122) of the inner packaging tube 12 for sensing by the probe 111. Therefore, a deflection angle of the flow direction of the blood in the process of reaching the sensing port 122 from the detection port 311 can be reduced and is less than 90°, so that a large-angle sudden change of the blood flow direction can be avoided, a blood flow pressure loss is effectively reduced, and detection precision of the sensor 11 is improved.

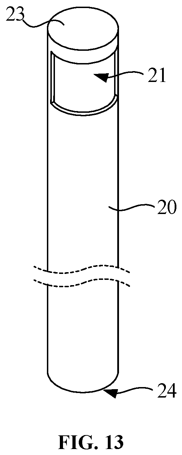

Referring to FIG. 8 and FIG. 13, optionally, in an embodiment, a proximal end of the outer packaging tube 20 is provided with an opening 24, so that the detection assembly 10 can be inserted into the outer packaging tube 20 through the opening 24 of the proximal end of the outer packaging tube 20, and the operation is simple. A distal end of the outer packaging tube 20 is optionally provided with a sealing structure 23, and the sealing structure 23 can seal the distal end of the outer packaging tube 20. As for a way of providing the flow guide wall 221 on the outer packaging tube 20, theoretically, the flow guide wall 221 can be molded together with the outer packaging tube 20 (that is, molded integrally with the outer packaging tube 20). However, considering that a diameter of the outer packaging tube 20 is small, and a mold for manufacturing the outer packaging tube 20 is also small, if the flow guide wall 221 is molded together with the outer packaging tube 20, the mold is modified, precision is difficult to control, manufacturing may be somewhat difficult, and a cost is relatively increased.

In view of this, a simpler way of providing the flow guide wall 221 on the outer packaging tube 20 is provided herein. Specifically, an inner side of the sealing structure 23 is filled with a third fixing adhesive 22, and a side wall of the third fixing adhesive 22 facing the distal tube opening 123 (i.e., the sensing port 122) of the inner packaging tube 12 defines the flow guide wall 221. During manufacturing, after the outer packaging tube 20 is formed, an adhesive solution may be dispensed from the side opening 21 of the outer packaging tube 20 to the inner side of the sealing structure 23, and during a curing process, the adhesive solution may spread towards the distal tube opening 123 of the inner packaging tube 12 under the influence of gravity (which is equivalent to spreading towards the lower right side of the view angle shown in FIG. 6), so that the third fixing adhesive 22 formed by curing the adhesive solution has a side wall extending obliquely towards the distal tube opening 123 of the inner packaging tube 12, and the side wall can be used as the flow guide wall 221. Therefore, the arrangement manner is simple to operate and low in cost.

In addition, since the sealing structure 23 is provided at the distal end of the outer packaging tube 20, the sealing structure 23 can avoid that the adhesive solution overflows from the distal end of the outer packaging tube 20 when the adhesive solution for forming the third fixing adhesive 22 is injected, and meanwhile, after the third fixing adhesive 22 is cured to form the flow guide wall 221, the third fixing adhesive 22 can be supported to enhance firmness of the third fixing adhesive 22 and prevented from loosening due to washing by the blood flow. Optionally, the third fixing adhesive 22 is a hard adhesive, so as to improve firmness and durability of the flow guide wall 221.

Referring to FIG. 8 and FIG. 9, in an embodiment, the flow guide wall 221 has an outer end adjacent to the distal edge 3111 and an inner end adjacent to the distal tube opening 123 (i.e., the sensing port 122). The flow guide wall 221 is arranged obliquely towards the distal tube opening 123 from the outer end to the inner end, so that after entering from the side opening 21 of the outer packaging tube 20, the blood can flow obliquely along the flow guide wall 221 towards the distal tube opening 123 of the outer packaging tube 12 and does not perpendicularly impact an inner wall surface of the outer packaging tube 20.

Further, the inner end of the flow guide wall 221 is axially spaced apart from the sensing port 122 by a first distance D₁. That is, the inner end of the flow guide wall 221 is not in contact with the distal tube opening 123 of the inner packaging tube 12. Since the flow guide wall 221 is formed by the third fixing adhesive 22, when the adhesive solution for forming the third fixing adhesive 22 is injected, if the adhesive solution contacts the distal tube opening 123 of the inner packaging tube 12, the adhesive solution may adhere to the sensor 11 at the distal tube opening 123 or a conductive wall 131 contacting the sensor 11, resulting in a decrease in sensitivity of the conductive wall 131 or the sensor 11 for sensing the blood pressure. Therefore, when the inner end of the flow guide wall 221 and the sensing port 122 are axially spaced apart by the first distance D₁, the adhesive solution for configuring the flow guide wall 221 can be prevented from adhering to the detection assembly 10, and then, the flow guide wall 221 is prevented from interfering with detection precision of the detection assembly 10.

Referring to FIG. 8 and FIG. 9, in an embodiment, if an inclination angle of the flow guide wall 221 relative to an axis of the outer packaging tube 20 is θ, then θₘᵢₙ≤θ<90°, where θₘᵢₙ represents a minimum limit value of the inclination angle θ. When θ=θₘᵢₙ, the inner end of the flow guide wall 221 just extends to the sensing port 122. Since the distal tube opening 123 of the inner packaging tube 12 forms the sensing port 122, optionally, the distal tube opening 123 of the inner packaging tube 12 is arranged on a bisecting plane AF which is a plane perpendicular to the axis of the motor 30 and passing through a bisector of an axial length of the side opening 21. In this case, it equivalently means that the distal tube opening 123 of the inner packaging tube 12 corresponds to the middle position of the axial length of the side opening 21, so that the detection assembly 10 can be seen from the side opening 21, and the outer packaging tube 20 can be conveniently bonded to the detection assembly 10 and the motor housing 30 at the side opening 21 for further fixing.

Since the distal tube opening 123 of the inner packaging tube 12 is arranged on the bisecting plane AF, if the side opening 21 has an axial length B, an axial distance between the distal tube opening 123 of the inner packaging tube 12 and the distal edge of the side opening 21 is 0.5B. Based on this, a relationship among the minimum limit value θₘᵢₙ, the axial length B of the side opening 21, and the inner diameter D₂ of the outer packaging tube 20 can be obtained according to a geometric principle: θₘᵢₙ=arctan(D₂/0.5B). That is, the relationships among the inclination angle θ of the flow guide wall 221, the axial length B of the side opening 21, and the inner diameter D₂ of the outer packaging tube 20 are θₘᵢₙ≤θ<90° and θₘᵢₙ=arctan(D₂/0.5B). It should be understood that, in the range of θₘᵢₙ≤θ<90°, the smaller the inclination angle θ, the better a flow guide effect of the flow guide wall 221. It should be further noted here that the side opening 21 of the outer packaging tube 20 has an equal size to the detection port 311 or a smaller size than the detection port 311.

In another embodiment, the distal tube opening 123 of the inner packaging tube 12 may not be located on the bisecting plane AF. In this case, the range of the inclination angle θ of the flow guide wall 221 relative to the axis of the outer packaging tube 20 may be selected to be 30°≤θ≤50°. The inclination angle θ may be, but is not limited to, 32°, 35°, 40°, 44° or 49°. Therefore, the flow guide wall 221 can be ensured to have a good flow guide effect, and the flow guide wall 221 is prevented from extending to the sensing port 122 to contact the detection assembly 10.

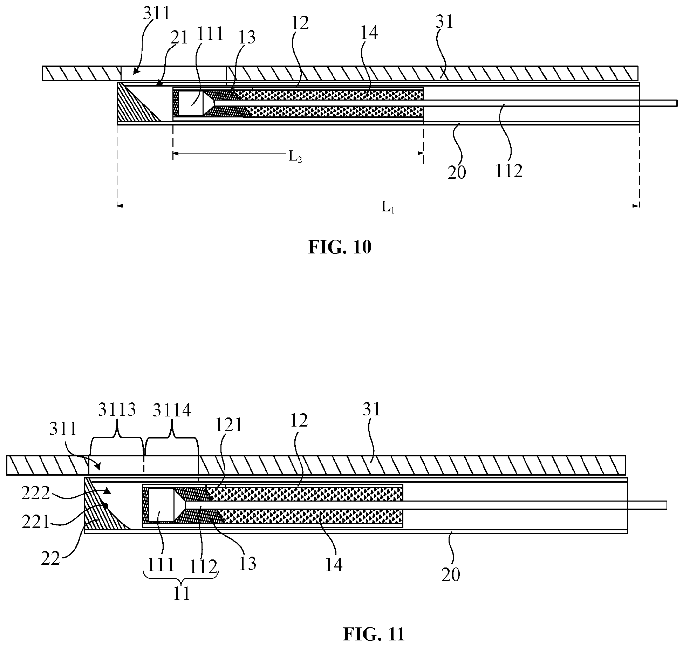

Referring to FIG. 9, optionally, in an embodiment, the flow guide wall 221 is configured as an inclined plane inclined relative to the axis of the outer packaging tube 20. Referring to FIG. 11, in another embodiment, the flow guide wall 221 may be configured as a concave arc surface that is recessed away from the distal tube opening 123 (i.e., the sensing port 122). Preferably, the flow guide wall 221 which is a concave arc surface may be formed by pressing the third fixing adhesive 22 with a tool having a spherical surface before the third fixing adhesive 22 is completely cured.

Referring to FIG. 9 and FIG. 10, a length of the outer packaging tube 20 is defined as L₁, a length of the inner packaging tube 12 is defined as L₂, and L₂<L₁. That is, the inner packaging tube 12 is shorter than the outer packaging tube 20, so that on the one hand, consumed materials can be reduced, and a cost is saved; on the other hand, under the condition of ensuring that an effect of fixing of the inner packaging tube 12 in the outer packaging tube 20 is good, an area of axial contact between the inner packaging tube 12 and the inner wall surface of the outer packaging tube 20 in the process of inserting the inner packaging tube 12 into the outer packaging tube 20 can be relatively reduced, resistance applied when the inner packaging tube 12 is inserted into the outer packaging tube 20 is then reduced, and therefore, the inner packaging tube 12 can be more easily inserted into the outer packaging tube 20. Optionally, 0.2L₁≤L₂≤0.5L₁.

Preferably, at least one of the outer packaging tube 20 and the inner packaging tube 12 is made of an organic polymer material. For example, the inner packaging tube 12 is made of the organic polymer material, so that the inner packaging tube 12 has certain ductility, and when an outer diameter of the inner packaging tube 12 is slightly greater than the inner diameter of the outer packaging tube 20, the inner packaging tube 12 can be stretched to slightly reduce the outer diameter of the inner packaging tube 12, so that the inner packaging tube 12 can be inserted into the outer packaging tube 20. Conversely, the inner packaging tube 12 can be expanded to increase an inner diameter and an outer diameter of the inner packaging tube 12.

Referring to FIG. 11, the detection port 311 includes an inlet region 3113 and a positioning region 3114, the inlet region 3113 corresponds to the flow guide groove 221 along a radial direction of the motor 30, and the positioning region 3114 corresponds to the distal end of the inner packaging tube 12 in the radial direction of the motor 30. A first adhesive is provided at the positioning region 3114, which can fixedly connect the inner packaging tube 12 to the outer packaging tube 20 and the motor housing 31. Further, the proximal end of the outer packaging tube 20 extends to a proximal end of the motor housing 31, and the proximal end of the outer packaging tube 20 is fixedly connected to the motor housing 31 by a second adhesive, so that connection stability of the outer packaging tube 20 and the motor housing 31 is enhanced at the proximal end of the outer packaging tube 20. Since the first adhesive and the second adhesive are both away from the probe 111, both the first adhesive and the second adhesive can be selected as hard adhesives.

Referring to FIG. 6 and FIG. 8, in an embodiment, the optical fiber 112 of the sensor 11 includes an inner optical fiber section 1122 and an outer optical fiber section 1121 which are connected. The inner optical fiber section 1122 is connected to the probe 111, and the inner optical fiber section 1122 and the probe 111 are both packaged in the inner packaging tube 12. The outer optical fiber section 1121 is connected to the inner optical fiber section 1122, and at least a part of the outer optical fiber section 1121 is received in the outer packaging tube 20. The outer optical fiber section 1121 may also be fixedly bonded to the outer packaging tube 20 and the motor housing 31 by the second adhesive at a position where the outer optical fiber section extends out from the proximal end of the outer packaging tube 20.

Referring to FIG. 7, optionally, in an embodiment, the inner packaging tube 12 is filled with a first fixing adhesive 13 and a second fixing adhesive 14. The first fixing adhesive 13 is filled in the distal end of the inner packaging tube 12, and the first fixing adhesive 13 wraps the probe 111. A part of the first fixing adhesive 13 forms the conductive wall 131 at the sensing port 122, and the conductive wall 131 is in contact with the probe 111 for transmitting the pressure. The second fixing adhesive 14 wraps at least a part of the optical fiber 112 in the inner packaging tube 12.

Specifically, the probe 111 of the sensor 11 is wrapped by the first fixing adhesive 13, and at least a part of the optical fiber 112 in the inner packaging tube 12 is wrapped by the second fixing adhesive 14. Thus, most of the sensor 11 can be sealed in the inner packaging tube 12 by the adhesive, so that the sensor 11 is firmly fixed in the inner packaging tube 12 and is not prone to slide in the axial direction of the inner packaging tube 12. Meanwhile, the part of the first fixing adhesive 13 forms the conductive wall 131 at the sensing port 122 of the inner packaging tube 12, the conductive wall 131 seals the sensing port 122, and the conductive wall 131 is in contact with the probe 111, so that the blood cannot enter the inner packaging tube 12 from the sensing port 122, while the blood pressure can be conducted to the probe 11 through the conductive wall 131, and the sensor 11 can be ensured to normally detect the blood pressure while the sensor 11 is fixed. In addition, the conductive wall 131 and the part of the first fixing adhesive 13 wrapping the probe 111 form an integral structure, so that structural stability of the conductive wall 131 is improved, the conductive wall 131 is not prone to loosen due to washing by the blood flow, and the detection precision of the sensor 11 is ensured. Preferably, the conductive wall 131 is coplanar with an end surface of the sensing port 122.

Referring to FIG. 7, in an embodiment, the first fixing adhesive 13 is a soft adhesive. Specifically, the probe 111 of the sensor 11 is a sensitive element, and the probe 111 is wrapped by the soft first fixing adhesive 13, so that the first fixing adhesive 13 can fix the probe 111 and perform buffering protection on the probe 111. In addition, the soft first fixing adhesive 13 has good elasticity, so that the conductive wall 131 formed by the part of the first fixing adhesive 13 is more sensitive to blood pressure changes, and can transmit the blood pressure to the probe 111 more precisely, thereby improving the detection precision of the sensor 11.

Referring to FIG. 7, in an embodiment, the second fixing adhesive 14 is a hard adhesive. Specifically, the optical fiber 112 of the sensor 11 is a non-sensitive element, and the second fixing adhesive 14 mainly plays a role of fixedly bonding the optical fiber 112 and an inner circumferential wall of the inner packaging tube 12 in the inner packaging tube 12, so that by configuring the second fixing adhesive 14 as a hard adhesive, adhesion and the firmness of the second fixing adhesive 14 are stronger, and the second fixing adhesive 14 has higher strength to fix and support the optical fiber 112. Certainly, in other embodiments, the second fixing adhesive 14 may be a soft adhesive on the premise of ensuring that the sensor 11 is not prone to loosen.

It should be understood that, the soft adhesive described herein means that the adhesive formed after the adhesive solution is solidified is an elastic body, such as silicon rubber and solvent glue; the hard adhesive means that the adhesive formed after the adhesive solution is cured is a hard inelastic body, such as an epoxy adhesive and a structural adhesive. That is, in the present application, the first fixing adhesive 13 may be selected as a soft adhesive; the second fixing adhesive 14 can be selected as a hard adhesive or a soft adhesive.

Referring to FIG. 6 and FIG. 8, further, the inner optical fiber section 1122 of the optical fiber 112 includes a first sub-section 1123 connected to the probe 111 and a second sub-section 1124 connected to the first sub-section 1123. The first fixing adhesive 13 also wraps the first sub-section 1123 and a joint of the first sub-section connected to the probe 111. The second fixing adhesive 14 wraps the second sub-section 1124. Preferably, the probe 111 and the first sub-section 1123 are welded, a welding spot is formed at a welding position of the probe 111 and the first sub-section 1123, and the first fixing adhesive 13 wraps the welding spot. Specifically, the probe 111 and the optical fiber 112 are generally fixedly welded through a welding material such as a tin material, the welding spot formed by hot melting of the welding material such as the tin material is fragile, and therefore, the welding spot of the probe 111 and the optical fiber 112 is wrapped by the soft first fixing adhesive 13, and the soft first fixing adhesive 13 can realize buffering protection of the welding spot, so that the second fixing adhesive 14 can be prevented from contacting the welding spot, and the problem that the welding spot is broken due to pressing of the welding spot after the second fixing adhesive 14 is cured is then avoided.

Further, one end of the first fixing adhesive 13 extends along the axial direction of the inner packaging tube 12 to be coplanar with the distal tube opening 123 of the inner packaging tube 12, the other end of the first fixing adhesive 13 is connected to the second fixing adhesive 14. An end of the second fixing adhesive 14 away from the first fixing adhesive 13 extends along the axial direction of the inner packaging tube 12 to be coplanar with the proximal tube opening 124 of the inner packaging tube 12. With this arrangement, the first fixing adhesive 13 and the second fixing adhesive 14 can cooperate to fill a gap in the inner packaging tube 12, so that the part of the sensor 11 located in the inner packaging tube 12 is wrapped and fixed, and fixing reliability of the sensor 11 can be improved; in addition, the tube openings at the two ends of the inner packaging tube 12 are also sealed, thus effectively improving the sealing performance of the sensor 11.

Referring to FIG. 6, FIG. 7 and FIG. 12, optionally, in an embodiment, an adhesive injecting hole 121 for injecting the second fixing adhesive 14 is formed in a side of the inner packaging tube 12. Referring to FIG. 7, the first fixing adhesive 13 is provided with a first inclined surface 132 on an inner side of the adhesive injecting hole 121, and the second fixing adhesive 14 is fixedly bonded to the first inclined surface 132. Specifically, in an embodiment, when the first fixing adhesive 13 and the second fixing adhesive 14 are injected into the inner packaging tube 12, the first fixing adhesive 13 may be injected through the distal tube opening 123 of the inner packaging tube 12 first, and the second fixing adhesive 14 is injected through the adhesive injecting hole 121 after the first fixing adhesive 13 is partially solidified.

Since the adhesive injecting hole 121 is formed in a side wall of the inner packaging tube 12, the first fixing adhesive 13 forms the first inclined surface 132 on the inner side of the adhesive injecting hole 121, and the first inclined surface 132 is inclined relative to the axial direction of the inner packaging tube 12, the first inclined surface 132 can guide the adhesive solution for forming the second fixing adhesive 14 towards the proximal end of the inner packaging tube 12, so that when applying the second fixing adhesive 14, the adhesive solution for forming the second fixing adhesive 14 can flow from the first inclined surface 132 towards the proximal tube opening 124 of the inner packaging tube 12, air in the inner packaging tube 12 is discharged from the proximal tube opening 124, formation of an air column between the first fixing adhesive 13 and the second fixing adhesive 14 is avoided, the first fixing adhesive 13 and the second fixing adhesive 14 can be ensured to completely fill the inner packaging tube 12, and a fixing effect on the sensor 11 is improved. Meanwhile, the first inclined surface 132 can be fixedly bonded to the cured second fixing adhesive 14, and compared with a vertical end surface, the first inclined surface 132 has a larger surface area, so that bonding strength of the first fixing adhesive 13 and the second fixing adhesive 14 can be enhanced.

Optionally, after the detection assembly 10 is inserted into the outer packaging tube 20, the adhesive injecting hole 121 in the side wall of the inner packaging tube 12 of the detection assembly 10 is covered by a side wall of the outer packaging tube 20, so as to prevent the adhesive injecting hole 121 from being exposed inside the side opening 21 of the outer packaging tube 20, reduce washing of the adhesive injecting hole 121 by the blood, and reduce the risk that the blood enters from the adhesive injecting hole 121.

Finally, it should also be noted that a sealing structure (such as a sealant) is usually further arranged between a periphery of the detection port 311 of the motor housing 31 and an outer circumferential wall of the outer packaging tube 20 to seal a gap between the periphery of the detection port 311 of the motor housing 31 and the outer circumferential wall of the outer packaging tube 20, so as to prevent the blood from entering the motor housing 31 through the gap, which is not repeated herein.

Referring to FIG. 1 to FIG. 3, in another aspect, the present application further provides a blood pump. Specifically, in an embodiment, the blood pump includes a catheter 40 and the driving device according to any of the above embodiments, and the catheter 40 is connected to the driving device. The optical fiber 112 of the sensor 11 extends through the catheter 40. Further, a control cable of the driving device, a flushing line, or the like, can be further arranged through the catheter 40, the control cable is configured to transmit an electrical signal, and the flushing line conveys an irrigation fluid or a lubricating fluid such as physiological saline and heparin to the driving device, so as to flush the driving device, which is not repeated herein.

In an embodiment, the blood pump is adapted to being arranged through the blood vessel of a patient into the ventricle of the patient to assist the ventricle in pumping blood into the blood vessel. The blood pump may further include a cannula assembly 71 and an impeller 72, the cannula assembly 71 is connected to a distal end of the driving device, and the cannula assembly 71 is provided with a blood outlet 711 and a blood inlet (not shown). The impeller 72 is arranged within the sleeve assembly 71 and connected to the driving device. The impeller 72 can be driven to rotate by the driving device to pump the blood. As an example, the blood pump is inserted into the left ventricle, and when the blood pump is inserted into the patient, the blood inlet of the blood pump is located in the left ventricle, and the blood outlet 711 of the blood pump is located in the aorta, so as to pump the blood in the left ventricle into the aorta.

Since the driving device according to this embodiment has a structure similar to that of the driving device according to the first embodiment, the driving device according to this embodiment and the blood pump having the driving device according to this embodiment also have effects similar to those of the first embodiment.

The above embodiments are only used to illustrate the technical solutions of the present invention, not to limit the present invention; although the present invention is described in detail with reference to the above embodiments, those having ordinary skill in the art should understand that they still can modify technical solutions recited in the aforesaid embodiments or equivalently replace partial technical features therein; these modifications or substitutions do not make essence of corresponding technical solutions depart from the spirit and scope of technical solutions of embodiments of the present invention, and all of them should be included in the protection scope of the present invention.

## Claims

1. A driving device, comprising:
a motor comprising a motor housing, and the motor housing being provided with a detection port;
an outer packaging tube inserted into the motor housing along an axial direction of the motor,
the outer packaging tube being fixedly connected to the motor housing, and a distal end of the outer packaging tube being provided with a side opening opposite to the detection port; and
a detection assembly comprising a sensor and an inner packaging tube, wherein the inner packaging tube is fixed inside the outer packaging tube, and a distal end of the inner packaging tube extends to an inner side of the side opening and is provided with a sensing port corresponding to the side opening; the sensor has a probe and an optical fiber connected to the probe, the probe and a part of the optical fiber are packaged in the inner packaging tube, and
the probe corresponds to the sensing port.

2. The driving device according to claim 1, wherein the detection port has a distal edge and a proximal edge opposite to each other; the inner packaging tube has a distal tube opening configured to form the sensing port, and the distal tube opening is located in an inner region between the distal edge and the proximal edge, the outer packaging tube is provided therein with a flow guide wall located on the inner side of the side opening, and a flow guide groove is formed between the flow guide wall and the distal tube opening.

3. The driving device according to claim 2, wherein the flow guide wall has an outer end adjacent to the distal edge and an inner end adjacent to the distal tube opening, the flow guide wall is inclined towards the distal tube opening from the outer end thereof to the inner end thereof, and the inner end of the flow guide wall is spaced apart from the distal tube opening by a first distance in the axial direction of the motor.

4. The driving device according to claim 2, wherein the flow guide wall is configured as an inclined plane inclined relative to an axis of the outer packaging tube;
or, the flow guide wall is configured as a concave arc surface recessed away from the distal tube opening.

5. The driving device according to claim 2, wherein the distal tube opening of the inner packaging tube is arranged on a bisecting plane, the bisecting plane being a plane perpendicular to an axis of the motor and passing through a bisector of an axial length of the side opening; wherein a relationship among an inclination angle θ of the flow guide wall relative to the axis of the motor, the axial length B of the side opening, and an inner diameter D₂ of the outer packaging tube is θₘᵢₙ≤θ<90°, wherein θₘᵢₙ=arctan(D₂/0.5B);
or, a range of the inclination angle θ of the flow guide wall relative to the axis of the motor is 30°≤θ≤50°.

6. The driving device according to claim 2, wherein a proximal end of the outer packaging tube is provided with an opening allowing the detection assembly to be inserted, the distal end of the outer packaging tube is provided with a sealing structure configured to seal the distal end of the outer packaging tube, an inner side of the sealing structure is filled with a third fixing adhesive, and a side wall of the third fixing adhesive facing the distal tube opening defines the flow guide wall.

7. The driving device according to claim 2, wherein the detection port comprises an inlet region and a positioning region, the inlet region corresponds to the flow guide groove along a radial direction of the motor; and the positioning region corresponds to the distal end of the inner packaging tube along the radial direction of the motor;
wherein a first adhesive is provided in the positioning region, and the first adhesive fixedly connects the inner packaging tube to the outer packaging tube and the motor housing;
and/or, the proximal end of the outer packaging tube extends to a proximal end of the motor housing and is fixedly connected to the motor housing through a second adhesive.

8. The driving device according to claim 1, wherein a length of the inner packaging tube is less than a length of the outer packaging tube;
wherein the optical fiber of the sensor comprises an inner optical fiber section and an outer optical fiber section that are connected, the inner optical fiber section is connected to the probe, the inner optical fiber section and the probe are packaged in the inner packaging tube, and the outer optical fiber section is connected to the inner optical fiber section and at least partially received in the outer packaging tube.

9. The driving device according to claim 1, wherein a length of the outer packaging tube is defined as L₁, and a length of the inner packaging tube is defined as L₂, and 0.2L₁≤L₂≤0.5L₁; and/or, at least one of the outer packaging tube and the inner packaging tube is made of an organic polymer material.

10. The driving device according to claim 1, wherein the inner packaging tube is filled with a first fixing adhesive and a second fixing adhesive, the first fixing adhesive is filled in a distal end of the inner packaging tube and wraps the probe, a part of the first fixing adhesive forms a conductive wall at the sensing port, the conductive wall is in contact with the probe; and the second fixing adhesive wraps at least a part of the optical fiber in the inner packaging tube.

11. The driving device according to claim 10, wherein the optical fiber comprises an inner optical fiber section located in the inner packaging tube, and the inner optical fiber section comprising a first sub-section connected to the probe and a second sub-section connected to the first sub-section, the first fixing adhesive also wraps the first sub-section and a joint of the first sub-section connected to the probe, and the second fixing adhesive wraps the second sub-section.

12. The driving device according to claim 10, further comprising at least one of the following features:
wherein the first fixing adhesive is a soft adhesive;
wherein the second fixing adhesive is a soft adhesive or a hard adhesive;
wherein one end of the first fixing adhesive extends along an axial direction of the inner packaging tube to be coplanar with a distal tube opening of the inner packaging tube;
wherein the second fixing adhesive is connected to the first fixing adhesive, and an end of the second fixing adhesive away from the first fixing adhesive extends along the axial direction of the inner packaging tube to be coplanar with a proximal tube opening of the inner packaging tube.

13. The driving device according to claim 10, wherein a side wall of the inner packaging tube is provided with an adhesive injecting hole for injecting the second fixing adhesive, the adhesive injecting hole is covered by a side wall of the outer packaging tube;
or, the first fixing adhesive forms a first inclined surface on an inner side of the adhesive injecting hole, the first inclined surface is inclined relative to an axial direction of the inner packaging tube, and the second fixing adhesive is fixedly bonded to the first inclined plane.

14. A blood pump, comprising:
a catheter; and
a driving device; wherein the driving device comprises:
a motor comprising a motor housing, and the motor housing being provided with a detection port;
an outer packaging tube inserted into the motor housing along an axial direction of the motor, the outer packaging tube being fixedly connected to the motor housing, and a distal end of the outer packaging tube being provided with a side opening opposite to the detection port; and
a detection assembly comprising a sensor and an inner packaging tube, wherein the inner packaging tube is fixed inside the outer packaging tube, and a distal end of the inner packaging tube extends to an inner side of the side opening and is provided with a sensing port corresponding to the side opening; the sensor has a probe and an optical fiber connected to the probe, the probe and a part of the optical fiber are packaged in the inner packaging tube, and the probe corresponds to the sensing port;
wherein the catheter is connected to the driving device, and the optical fiber of the sensor penetrates out of the catheter.

15. The blood pump according to claim 14, wherein the inner packaging tube is filled with a first fixing adhesive and a second fixing adhesive, the first fixing adhesive is filled in a distal end of the inner packaging tube and wraps the probe, a part of the first fixing adhesive forms a conductive wall at the sensing port, and the conductive wall is in contact with the probe; and the second fixing adhesive wraps at least a part of the optical fiber in the inner packaging tube.

16. The blood pump according to claim 15, wherein the optical fiber comprises an inner optical fiber section located in the inner packaging tube, and the inner optical fiber section comprising a first sub-section connected to the probe and a second sub-section connected to the first sub-section, the first fixing adhesive also wraps the first sub-section and a joint of the first sub-section connected to the probe, and the second fixing adhesive wraps the second sub-section.

17. The blood pump according to claim 16, wherein the driving device further comprises at least one of the following features:
the first fixing adhesive is a soft adhesive;
the second fixing adhesive is a hard adhesive;
one end of the first fixing adhesive extends along an axial direction of the inner packaging tube to be coplanar with a distal tube opening of the inner packaging tube;
the second fixing adhesive is connected to the first fixing adhesive, and an end of the second fixing adhesive away from the first fixing adhesive extends along the axial direction of the inner packaging tube to be coplanar with a proximal tube opening of the inner packaging tube.

18. The blood pump according to claim 15, wherein a side wall of the inner packaging tube is provided with an adhesive injecting hole for injecting the second fixing adhesive, the adhesive injecting hole is covered by a side wall of the outer packaging tube;
or, the first fixing adhesive forms a first inclined surface on an inner side of the adhesive injecting hole, the first inclined surface is inclined relative to an axial direction of the inner packaging tube, and the second fixing adhesive is fixedly bonded to the first inclined plane.

19. The blood pump according to claim 14, wherein the detection port has a distal edge and a proximal edge opposite to each other; the inner packaging tube has a distal tube opening configured to form the sensing port, and the distal tube opening is located in an inner region between the distal edge and the proximal edge, the outer packaging tube is provided therein with a flow guide wall located on the inner side of the side opening, and a flow guide groove is formed between the flow guide wall and the distal tube opening.

20. The blood pump according to claim 14, further comprising:
a cannula assembly connected to an end of the driving device away from the catheter, and the cannula assembly being provided with a blood inlet and a blood outlet; and
an impeller arranged in the cannula assembly and connected to the driving device, and the impeller being driven to rotate by the driving device.
